# EUROPEAN PATENT APPLICATION

(11) **EP 1 913 941 A1**
(43) Date of publication of application: **23.04.2008**
(21) Application number: 06122602.3
(22) Date of filing: 19.10.2006
(51) Int. Cl.: A61K 31/137, A61P 5/14, C07C 211/30

(54) **Polymorphic forms and solvates of Cinacalcet hydrochloride**

(71) Applicant: SANDOZ AG, 4056 Basel (CH)
(72) Inventor: The designation of the inventor has not yet been filed
(74) Representative: Fauss-Berghus, Waltraud

(57) **Abstract**

The present invention relates to crystalline forms of Cinacalcet hydrochloride, methods for the preparation thereof, the use of crystalline forms of Cinacalcet hydrochloride in the treatment of of hyperparathyroidism and to pharmaceutical compositions comprising crystalline forms of Cinacalcet hydrochloride.

The present invention also relates to novel solvates of Cinacalcet Hydrochloride, methods for the preparation of these solvates, the use of these novel solvates for the preparation of pure Cinacalcet Hydrochloride, the use of these novel solvates for the preparation of polymorphic form II of Cinacalcet Hydrochloride and pharmaceutical compositions comprising the acetic acid solvate of Cinacalcet Hydrochloride.

## Description

The present invention relates to new polymorphic forms of Cinacalcet hydrochloride, methods for the preparation thereof, the use of these new polymorphic forms of Cinacalcet hydrochloride in the treatment of hyperparathyroidism and of hypercalcemia and to pharmaceutical compositions comprising the new polymorphic forms of Cinacalcet hydrochloride.

Cinacalcet hydrochloride, N-[1-(R)-(-)-(1-naphthyl)ethyl]-3-[3-(trifluoromethyl)phenyl]-1-aminopropane hydrochloride, shown as Compound (I) below 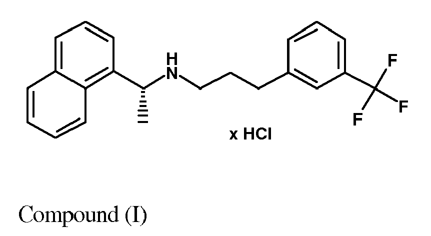 is a novel second generation calcimimetic that modulates the extra cellular calcium sensing receptor (CaR) by making it more sensitive to the calcium suppressive effects on parathyroid hormone (PTH). It is used in a treatment for primary and secondary hyperparathyroidism. Hyperparathyroidism is characterized by high levels of circulating calcium due to an increased secretion of parathyroid hormone by one or more of the parathyroid glands. Hyperparathyroidism can lead to e.g. osteoporosis; patients with renal failure suffering from secondary hyperparathyroidism have for example an increased risked of renal bone disease, soft-tissue calcifications and vascular disease.

The preparation of Cinacalcet is, for example, described in Drugs of the Future 2002,27(9),831-836 and its use in the treatment of primary and secondary hyperparathyroidism has been the subject of several research articles, e.g. Expert opinion on investigational drugs (2003), 12(8), 1413-21.

Cinacalcet is sold e.g. in US as Sensipar® in the form of tablets. Sensipar® is to be used in the treatment of hyperparathyroidism and of hypercalcemia.

The isolation of Cinacalcet as hydrochloride is not described in the patent literature. US 6211244 examplifies the synthesis and isolation of analogues. Hydrochlorides of these analogues are prepared by the precipitation using HCl g in ether or hexane in combination with HCl g in ether. This method is not applicable to large scale synthesis.

Polymorphism is a phenomenon relating to the occurrence of different crystal forms for one molecule. There may be several different crystalline forms for the same molecule with distinct crystal structures and varying in physical properties like melting point, XRPD spectrum and IR-spectrum. These polymorphs are thus distinct solid forms which share the molecular formula of the compound from which the crystals are made up, however they may have distinct advantageous physical properties which can have a direct effect on the ability to process and/or manufacture the drug substance, like flowability, and the drug product, like flowability, as well as on drug product stability, dissolution, and bioavailability.

There is thus a need for new crystalline forms of cinacalcet hydrochloride and for processes for the production of such crystalline forms.

### Summary of the invention

The present invention therefore relates to the polymorph form II of Cinacalcet hydrochloride, the polymorph form III of Cinacalcet hydrochloride, methods for the preparation of pure form II of Cinacalcet hydrochloride, methods for the preparation of pure form III of Cinacalcet hydrochloride, and pharmaceutical compositions comprising the new polymorphic forms in an pharmaceutically effective amount.

The present invention also provides novel solvates of Cinacalcet Hydrochloride, methods for the preparation of these solvates, the use of these novel solvates for the preparation of pure Cinacalcet Hydrochloride, the use of these novel solvates for the preparation of polymorphic form II of Cinacalcet Hydrochloride and pharmaceutical compositions comprising the acetic acid solvate of Cinacalcet Hydrochloride.

### Detailed description of the invention

The present inventors have surprisingly identified new polymorphic forms of Cinacalcet Hydrochloride which are stable upon storage. This property is important and proves advantageous for the desired use of Cinacalcet Hydrochloride in pharmaceutical formulations.

The invention therefore relates in a first embodiment to polymorph form II of Cinacalcet hydrochloride.

In a preferred embodiment the invention relates to a crystalline form II of Cinacalcet hydrochloride with a PXRD (X-ray powder diffraction pattern) comprising peaks at 2 Theta angles of 6.8 ±0.2°, 12.3 ±0.2°, 13.7 ±0.2°, 16.4 ±0.2°, 17.5 ±0.2, 19.4 ±0.2, 20.3 ±0.2 and 23.3 ±0.2, in particular of 6.79±0.1, 12.26 ±0.1 13.65 ±0.1°, 16.38 ±0.1, 17.50 ±0.1, 19.42 ±0.1, 20.27 ±0.1 and 23.32 ±0.1.

In an alternative preferred embodiment the invention relates to a crystalline form II of cinacalcet hydrochloride which shows, when analyzed by DSC (differential scanning calorimetry), a peak between 168°C and 175°C, preferably at about 170°C, followed by a further peak at between 176°C and 183°C, preferably at about 180°C.

In a very preferred embodiment, the invention relates to a crystalline form II of Cinacalcet hydrochloride with a PXRD comprising peaks at 2 Theta angles of 16.4 ±0.2°, and 19.4 ±0.2, and showing a peak in a DSC at between 168°C and 175°C, in particular a crystalline form II of Cinacalcet hydrochloride with a PXRD spectrum comprising peaks at 2 Theta angles of 6.8 ±0.2°, 12.3 ±0.2°, 13.7 ±0.2°, 16.4 0.2°, 17.5 ±0.2, 19.4 ±0.2, 20.3 ±0.2 and 23.3 ±0.2 and showing a peak in a DSC between 168°C and 175°C, preferably at about 170°C, followed by a further peak at between 176°C and 183°C.

Form II may be prepared by desolvation of solvates of Cinacalcet hydrochloride at a temperature of about 50°C to about 150°C, preferably at temperatures of about 110 ° to 140°C. Form II may be also obtained from amorphous Cinacalcet hydrochloride at temperatures of above about 130°C. Preparation of form II is described in detail in example 8.

The invention further relates to the use of Cinacalcet hydrochloride form II for the production of a pharmaceutical composition. The invention further relates to a pharmaceutical composition comprising Cinacalcet hydrochloride form II, preferably in a pharmaceutically effective amount.

Preferred pharmaceutical compositions of the invention are oral dosage forms such as tablets, capsules, powders for oral suspension, pills and granules. For example Cinacalcet hydrochloride form II of the invention can be formulated as tablets for oral administration comprising from 20mg to 300mg and in particular from 30mg to 120mg Cinacalcet Hydrochloride, and further comprising pre-gelatinized starch, microcrystalline cellulose, povidone, crospovidone, colloidal silicon dioxide and magnesium stearate, preferably in amounts equivalent to the marketed product Sensipar® as sold in the US on the priority date. Preferably the tablets are also coated with color, clear film coat and/or carnauba wax.

The invention further relates to a method of treating primary and secondary hyperparathyroidism in a mammal comprising using Cinacalcet hydrochloride form II. The invention further relates to the use of Cinacalcet hydrochloride form II in the preparation of a medicament for the treatment of hyperparathyroidism, in particular for the prevention of treatment of osteoporosis, increased risked of renal bone disease, soft-tissue calcifications and vascular disease associated with hyperparathyroidism

The invention further relates to a pharmaceutical composition wherein 90% by weight of the comprised Cinacalcet hydrochloride is in the form of Cinacalcet hydrochloride form II, preferably wherein 95% by weight is in the form of Cinacalcet hydrochloride form II, and more preferably 99% are Cinacalcet hydrochloride form II. Most preferably the invention relates to pure isolated Cinacalcet hydrochloride form II and a pharmaceutical composition comprising pure isolated Cinacalcet hydrochloride form II as the only crystalline form of Cinacalcet hydrochloride, more preferably as the only form of Cinacalcet hydrochloride.

The present inventors have identified a further polymorphic form of Cinacalcet hydrochloride, designated form III below.

The invention therefore further relates to polymorph form III of Cinacalcet hydrochloride.

In a preferred embodiment the invention relates to a crystalline form III of Cinacalcet hydrochloride with a PXRD (X-ray powder diffraction pattern) comprising peaks at 2 Theta angles of 6.9±0.2°, 10.3 ±0.2°, 13.8 ±0.2°, 19.0 ±0.2, 20.8 ±0.2, 21.2 ±0.2, 24.2 and 25.4 ±0.2, in particular comprising peaks at 2 Theta angles of 6.86±0.1°, 10.35 ±0.1°, 13.80 ±0.1°, 18.97 ±0.1, 20.79 ±0.1, 21.23 ±0.1, 24.22 and 25.44 ±0.1.

Form III of cinacalcet hydrochloride may also be characterized in that it shows, when analyzed by DSC (differential scanning calorimetry), a peak between 163°C and 168°C, preferably a peak giving an onset temperature at about 164°C, followed by a further peak at between 176°C and 183°C, preferably at about 180°C.

Alternatively, form III of cinacalcet hydrochloride may also be characterized by a PXRD comprising peaks at 2 Theta angles of 19.0±0.2°, and 21.2 ±0.2, and showing a peak in a DSC at between 163°C and 168°C, in particular a crystalline form III of Cinacalc et hydrochloride with a PXRD spectrum comprising peaks at 2 Theta angles of 6.9 ±0.2°, 10.3 ±0.2°, 13.8 ±0.2°, 19.0 ±0.2, 20.8 ±0.2, 21.2 ±0.2, 24.2 and 25.4 ±0.2 and showing a peak in a DSC between 163°C and 168°C, preferably giving an onset temperature of about 164°C, followed by a further peak at between 176°C and 183°C, preferably at about 180°C. Alternatively, form III of cinacalcet hydrochloride may also be characterized by the unit cell of the crystals determined at 173K. The crystals belong to an orthorhombic crystal system of space group P2₁2₁2₁. The unit cell preferably is characterized by a cell volume of 2055 ? ³to 2065 ? ³, a value for the lattice constant a of 7.17 ± 0.04 ? , for b of 11.35 ± 0.05 ? and for c of 25.31 ± 0.04 ? , a value for Z of 4.

A preferred physical form of the crystalline form III of Cinacalcet hydrochloride of the invention are form III crystals in the form of plates. A plate-shaped crystal as used herein is a crystal wherein the ratio of the two larger dimensions of the crystal is from 0.1 to 10, preferably from 0.2 to 5. The preferred processes for the preparation of crystalline form III of Cinacalcet Hydrochloride of the present invention, in particular crystallization in the presence of the preferred protic solvents, provide a composition of crystalline Cinacalcet hydrochloride form III wherein more than 60% of the analyzed crystals are in the form of plates, preferably more than 80%, more preferably more than 90%. Such compositions are particularly easy to handle during pharmaceutical formulation of Cincalcet hydrochloride and provide an important advantage.

The invention further relates to the use of Cinacalcet hydrochloride form III of the invention for the production of a pharmaceutical composition. The invention further relates to the use of the composition of crystalline Cinacalcet hydrochloride form III wherein more than 60% of the analyzed crystals of Cinacalcet hydrochloride are in the form of plates of the invention for the production of a pharmaceutical composition. The invention further relates to a pharmaceutical composition comprising Cinacalcet hydrochloride form III, preferably wherein Cinacalcet hydrochloride form III is present in a pharmaceutically effective amount. The invention further relates to a pharmaceutical composition comprising the composition of crystalline Cinacalcet hydrochloride form III wherein more than 60% of the analyzed crystals of Cinacalcet hydrochloride are in the form of plates of the invention. The invention further relates to a pharmaceutical composition wherein 90% by weight of the comprised Cinacalcet hydrochloride is in the form of Cinacalcet hydrochloride form III, preferably wherein 95% is Cinacalcet hydrochloride form III, more preferably wherein 99% is Cinacalcet hydrochloride form III. Most preferably the invention relates to pure isolated Cinacalcet hydrochloride form III and a pharmaceutical composition comprising pure isolated Cinacalcet hydrochloride form III as the only crystalline form of Cinacalcet hydrochloride, preferably as the only form of Cinacalcet hydrochloride.

The invention further relates to the use of Cinacalcet hydrochloride form III for the production of a pharmaceutical composition. The invention further relates to a pharmaceutical composition comprising Cinacalcet hydrochloride form III, preferably in a pharmaceutically effective amount.

Preferred pharmaceutical compositions of the invention are oral dosage forms such as tablets, capsules, powders for oral suspension, pills and granules. For example Cinacalc et hydrochloride form III of the invention can be formulated as tablets for oral administration comprising from 20mg to 300mg and in particular from 30mg to 120mg Cinacalcet Hydrochloride, and further comprising pre-gelatinized starch, microcrystalline cellulose, povidone, crospovidone, colloidal silicon dioxide and magnesium stearate, preferably in amounts equivalent to the marketed product Sensipar® as sold in the US on the priority date. Preferably the tablets are also coated with color, clear film coat and/or carnauba wax.

The invention further relates to a method of treating primary and secondary hyperparathyroidism in a mammal comprising using Cinacalcet hydrochloride form III. The invention further relates to the use of Cinacalcet hydrochloride form III in the preparation of a medicament for the treatment of hyperparathyroidism, in particular for the prevention of treatment of osteoporosis, increased risked of renal bone disease, soft-tissue calcifications and vascular disease associated with hyperparathyroidism Polymorph purity of form III of Cinacalcet hydrochloride may be determined by IR, XRD or DSC.

The preferred method for the determination of polymorph purity is DSC, especially when considering polymorph purity with respect to polymorph II of Cinacalcet hydrochloride. In particular, the presence of more than 0,5% form II can be detected by DSC, e.g. by the detectable onset of form II of Cinacalcet hydrochloride at about 170°C.

The invention further relates to a process for the preparation of pure form III of Cinacalcet hydrochloride comprising providing a solution of Cinacalcet hydrochloride in a solvent selected from the list consisiting of acetone, methylethylketone, methylisobutylketone, 1-butanol, 2-butanol, ethanol, 3-methyl-1-butanol, isobutylalcohol, n-propanol, 2-propanol, 1-pentanol, butylacetate, ethylacetate, ethylformate, isobutylacetate, isopropylacetate, methylacetate, propylacetate, t-butylmethylether, diethylether, diisopropylether, anisol, dimethylsulfoxide, N-methylpyrrolidone, acetonitrile, dichloromethane, 1,2-dimethoxyethane, N,N-dimethylacetamide, N,N-dimethylformamide, n-hexane, methylcyclohexane, methanole, 2-methoxyethanole, 2-ethoxyethanole, methylbutylketone, xylene, 1,1-diethoxypropane and 1,1-dimethoxymethane, and preferably selected from the list consisting of acetone, methylethylketone, methylisobutylketone, 1-butanol, 2-butanol, ethanol, 3-methyl-1-butanol, isobutylalcohol, n-propanol, 2-propanol, 1-pentanol, butylacetate, ethylacetate, ethylformate, isobutylacetate, isopropylacetate, methylacetate, propylacetate, t-butylmethylether, diethylether, diisopropylether, anisol, dimethylsulfoxide, and N-methylpyrrolidone, mixtures of these solvents or mixtures of these solvents,with , n-hexane, methylcyclohexane, n-pentane,cyclohexane or water or mixtures of formic acid or acetic acid or tetrahydrofurane and water, with the proviso that dioxane is not to be used as single solvent or in combination with an alkane,
crystallization of pure form III of Cinacalcet hydrochloride from the obtained solution of Cinacalcet hydrochloride, for example by cooling or partially concentrating the solution, and isolating the pure crystalline form III of Cinacalcet hydrochloride. Crystallization in the presence of protic solvents tends to give plate-shaped Form III of Cinacalcet hydrochloride, while crystallization in the absence of protic solvents, like in the presence of aprotic solvents only, tends to give the less favoured needle-like Form III of Cinacalcet Hydrochloride.

Thus, in a preferred embodiment, the invention relates to a process for the preparation of pure form III of Cinacalcet hydrochloride in the form of plates comprising providing a solution of Cinacalcet hydrochloride in a solvent selected from the list consisiting of a mixture of acetone and hexane, acetonitrile, n-butanole, a mixture of n-butanole and hexane, dioxane, dimethylformamide, dimethylsulfoxide, ethylmethylketone, ethanole, methanole, nitromethane, n-propanole, water and pyridine, or mixtures thereof, and preferably in a solvent selected from the list consisiting of acetonitrile, a mixture of n-butanole and hexane, dioxane, dimethylformamide, dimethylsulfoxide, ethylmethylketone, ethanole, methanole, nitromethane and n-propanole, or mixtures of these with water, with the proviso that dioxane is not used as single solvent or in combination with an alkane, and most preferably in a solvent selected from the list consisiting of ethylmethylketone, ethanole, and n-propanole, or mixtures of these with water, crystallization of pure form III of Cinacalcet hydrochloride in the form of plates from the obtained solution of Cinacalcet hydrochloride, for example by cooling or partially concentrating the solution, and isolating the pure crystalline form III of Cinacalcet hydrochloride in the form of plates.

For both the process for the preparation of Cinacalcet hydrochloride form III and the process for the preparation of Cinacalcet hydrochloride form III in the form of plates, it is preferred that a saturated solution of Cinacalcet hydrochloride be prepared. A saturated solution can, for example, be prepared by dissolution of Cinacalcet hydrochloride in the solvent or the solvent mixture from about ambient temperature to the boiling point of the solution. Crystallization may then be induced by cooling of the solution. Seeds may be present in the crystallization procedure. The cooling procedure may be performed by cooling the solution or suspension, preferably to a temperature of about -20°C to about 10°C, more preferably to a temperature of between about -20°C and about 0°C. Preferably cooling is performed slowly, e.g. within several hours or e.g. within approximately 10 to 120 min.

The pure form III of Cinacalcet Hydrochloride, preferably in the form of plates, may be isolated by conventional methods, e.g. filtration and dried by conventional methods, e.g. air drying, drying with under a flow of nitrogen , or vaccum drying.

Pure form III of Cinacalcet hydrochloride may also be crystallized by providing a solution of Cinacalcet hydrochloride in a suitable solvent or solvent mixture followed by an addition, preferably a slow addition, of an antisolvent to the solution. Optionally seeds of form III may be added to induce crystallization. The ratio of solvent to antisolvent may vary from approximately a ratio of 1: 0.2 to a ratio of 1:20, preferably a ratio of about 1:1 to about 1:5; preferably 1:2 is used. Temperature may vary from about ambient temperature to the boiling point of the solvent, preferable from about ambient temperature to about 100°C. Cooling may be applied after the addition of the antisolvent if appropriate. An antisolvent is a solvent or solvent-system where cinacalcet form III shows reduced solubility as compared to its solubility in the solvent or solvent system. The antisolvent is typically an alkane, e.g. hexane, or an aqueous solution, preferably water. Cinacalcet free base may be prepared by methods know in the literature, e.g. by reductive amination of 3-(3-trifluoromethyl-phenyl)-propionadehyde with 1 (R) - (1-naphthylethylamine as disclosed e.g. in Drugs of the future 2002, 27(99), 831-836. Alternatively Cinacalcet hydrochloride or a salt of Cinacalcet with an organic acid or inorganic acid may be used as starting material. A solution of these salts may be used directly as starting material for hydrochloride formation described below or these salts may be converted to the free base, e.g. by means of neutralization of a solution of these salts with a suitable base.

The solution of Cincalcet hydrochloride may be provided by mixing of Cinacalcet free base with a hydrochloride source, e.g. aqueous or gaseous HCl, e.g. in stoichiometric amounts or using an excess of the hydrochloride source, e.g. up to 5 equivalents of the hydrochloride source in a solvent or solvent mixture as described above. A preferred way to generate Cinacalcet hydrochloride is the use of a trialkylsilylchloride in combination with a protic solvent as hydrochloride source as described in detail in Co-pending European application EP06116134, herein incorporated by reference. A very preferred process for the preparation of a solution of Cinacalcet hydrochloride comprising the steps of:
(a) dissolving the free base of Cinacalcet in a protic solvent, and
(b) adding a trialkylsilylchloride, preferably trimethylchlorosilane, in an amount of about one mole equivalent calculated based on Cinacalcet free base. The resulting solution, for example in ethanole, may be used as starting material for the above described processes for the preparation of Cinacalcet form III. The present invention also relates to mixtures of form III and form II of Cinacalcet hydrochloride.

Pure Cinacalcet hydrochloride form III as used herein relates to crystalline Cinacalcet hydrochloride having less than 20%, preferably less than 10%, more preferably less than 5 %, even more preferably less than 1%, and most preferably less than 0.5% of Cinacalcet hydrochloride form II present. 0.5%

Cinacalcet hydrochloride form II are still barely detectable when measured by DSC.

Form III of Cinacalcet hydrochloride is stable at least for 3 months when stored at ambient temperature Form III of Cinacalcet hydrochloride in the form of plates displays favourable flowability characteristics and allows for an overall easy handling.

The present inventors have also surprisingly identified novel solvates of Cinacalcet Hydrochloride which allow improved purification of Cinacalcet Hydrochloride and also the preparation of novel polymorphic form II of Cinacalcet Hydrochloride.

The invention therefore also relates to solvates of Cinacalcet Hydrochloride with certain small organic compounds comprising one or two consecutive carbon atoms, in particular wherein the solvates are crystalline and more particularly wherein the ratio of small organic compound to Cinacalcet Hydrochloride is between 0.5 and 2.0, in particular between 0.7 and 1.3, more particularly between 0.8 and 1.2 and most preferably about equimolar. Small organic compounds have a molecular weight below 250 Da, in particular below 150 Da.

In a preferred embodiment, the invention relates to a solvate of Cinacalcet Hydrochloride with Dioxane, in particular wherein the solvate is crystalline. The Cinacalcet Hydrochloride solvate with Dioxane of the invention preferably shows a ratio of Dioxane to Cinacalcet Hydrochloride of between 0.5 and 2.0, in particular of between 0.7 and 1.3, more particularly of between 0.8 and 1.1 and most preferably of about 0.9.

Alternatively, the Cinacalcet Hydrochloride solvate with Dioxane of the invention, when analyzed by PXRD, can be characterized by displaying a spectrum comprising peaks at 2 theta angles of about 6.5 ± 0.2°, 13.0 ± 0.2°, 14.8 ± 0.2°, 16.5 ± 0.2°, 19.4 ± 0.2°, 19.6± 0.2°.

Alternatively, the Cinacalcet Hydrochloride solvate with Dioxane of the invention, when analyzed by IR, can be characterized by displaying a spectrum comprising peaks at about 2963, 2853, 2797, 2752, 2715, 1451 1327, 1168, 1123, 1073, 873, 799, 779 and 706 cm-1.

In a preferred embodiment, the invention relates to a solvate of Cinacalcet Hydrochloride with Dioxane having a ratio of Dioxane to Cinacalcet Hydrochloride of between 0.8 and 1.1, and comprising peaks in a PXRD spectrum at 2 theta angles of 14.8 ± 0.2° and 19.4 ± 0.2° and preferably also at 6.5 ± 0.2° and 19.6± 0.2°.

The solvate with dioxane may be prepared e.g. from a hot saturated solution in dioxane, optionally in the presence of a co solvents, e.g. an alkane, e.g. hexane or heptane.

The dioxane solvate of Cinacalcet Hydrochloride can be used for the purification of Cinacalcet Hydrochloride and for the preparation of a new polymorphic form II of Cinacalcet hydrochloride, for example at temperatures of greater than 110°C by desolvation.

In a further preferred embodiment, the invention relates to a solvate of Cinacalcet Hydrochloride with acetic acid, in particular wherein the solvate is crystalline.

The Cinacalcet Hydrochloride solvate with acetic acid of the invention preferably shows a ratio of acetic acid to Cinacalcet Hydrochloride of between 0.5 and 2.0, in particular of between 0.7 and 1.4, more particularly of between 0.9 and 1.1 and most preferably of about equimolar.

Alternatively, the Cinacalcet Hydrochloride solvate with acetic acid of the invention, when analyzed by PXRD, displays a spectrum comprising peaks at 2 theta angles of about 15.1 ± 0.2°, 17.6 ± 0.2°, 18.3 ± 0.2°, 18.9 ± 0.2°, 19.6 ± 0.2°, 20.9 ± 0.2°, 24.7 ± 0.2°.

Alternatively, the Cinacalcet Hydrochloride solvate with acetic acid of the invention, when analyzed by IR, displays a spectrum with characteristic bands at about 2969, 2815, 2761, 1452,1329,1220, 1166,1122,1073, 807,795, 776 and 707 cm-1.

In a preferred embodiment, the invention relates to a solvate of Cinacalcet Hydrochloride with acetic acid having a ratio of acetic acid to Cinacalcet Hydrochloride of between 0.8 and 1.2, and comprising peaks in a PXRD spectrum at 2 theta angles of 18.9 ± 0.2° and 19.6 ± 0.2°, and preferably also at 15.1 ± 0.2°, 18.3 ± 0.2° and 24.7 ± 0.2°.

The solvate with acetic acid may be prepared e.g. by crystallization from a hot saturated solution. The solution may be then cooled, e.g. to a temperature of about ambient temperature and about 5°C.

The acetic acid solvate of Cinacalcet Hydrochloride can be used for the purification of Cinacalcet Hydrochloride and for the preparation of new polymorphic form II of Cinacalcet hydrochloride, for example by drying of the solvate at about 140°C.

The present invention also relates to a pharmaceutical composition comprising the Cinacalcet Hydrochloride solvate with acetic acid of the invention and a suitable excipient.

Preferred pharmaceutical compositions of the invention are oral dosage forms such as tablets, capsules, powders for oral suspension, pills and granules. For example the the Cinacalcet Hydrochloride solvate with acetic acid of the invention can be formulated as tablets for oral administration comprising from 20mg to 300mg and in particular from 30mg to 120mg Cinacalcet Hydrochloride, and further comprising pre-gelatinized starch, microcrystalline cellulose, povidone, crospovidone, colloidal silicon dioxide and magnesium stearate, preferably in amounts equivalent to the marketed product Sensipar® as sold in the US on the priority date. Preferably the tablets are also coated with color, clear film coat and/or carnauba wax.

The invention further relates to a method of treating primary and secondary hyperparathyroidism in a mammal comprising using the Cinacalcet Hydrochloride solvate with acetic acid of the invention. The invention further relates to the use of the Cinacalcet Hydrochloride solvate with acetic acid of the invention in the preparation of a medicament for the treatment of hyperparathyroidism, in particular for the prevention of treatment of osteoporosis, increased risked of renal bone disease, soft-tissue calcifications and vascular disease associated with hyperparathyroidism

In a further preferred embodiment the solvate of Cinacalcet Hydrochloride is a solvate with Chloroform (CHCl₃) or a solvate with tetrachloromethane (CCl₄), in particular wherein the solvate is crystalline.

In a further preferred embodiment, the invention relates to a solvate of Cinacalc et Hydrochloride with chloroform, in particular wherein the solvate is crystalline.

The Cinacalcet Hydrochloride solvate with chloroform of the invention preferably shows a ratio of chloroform to Cinacalcet Hydrochloride of between 0.5 and 2.0, in particular of between 0.7 and 1.4, more particularly of between 0.9 and 1.1 and most preferably of about equimolar.

Alternatively, the Cinacalcet Hydrochloride solvate with chloroform of the invention, when analyzed by PXRD, displays a spectrum comprising peaks at 2 theta angles of about 16.3 ± 0.2°, 17.9 ± 0.2, 20.3 ± 0.2 , 21.5 ± 0.2, 24.7 ± 0.2, 27.8 ± 0.2°.

Alternatively, the Cinacalcet Hydrochloride solvate with chloroform of the invention, when analyzed by IR, displays a spectrum with characteristic bands at about 2965, 2799, 2755, 2713, 1452, 1328, 1165, 1128, 1074, 799, 778, 750, and 704 cm-1.

In a preferred embodiment, the invention relates to a solvate of Cinacalcet Hydrochloride with chloroform having a ratio of chloroform to Cinacalcet Hydrochloride of between 0.8 and 1.2, and comprising peaks in a PXRD spectrum at 2 theta angles of 16.3 ± 0.2° and 21.5 ± 0.2, and preferably also at 24.7 ± 0.2.

The solvate with chloroform may be prepared e.g. from a hot saturated solution in chloroform.

The chloroform solvate of Cinacalcet Hydrochloride can be used for the purification of Cinacalcet Hydrochloride and for the preparation of new polymorphic form II of Cinacalcet hydrochloride, for example by desolvation of the solvate at about 110°C.

In a further preferred embodiment, the invention relates to a solvate of Cinacalcet Hydrochloride with tetrachloromethane, in particular wherein the solvate is crystalline.

The Cinacalcet Hydrochloride solvate with terachloromethane of the invention preferably shows a ratio of tetrachloromethane to Cinacalcet Hydrochloride of between 0.5 and 2.0, in particular of between 0.7 and 1.3, more particularly of between 0.8 and 1.1 and most preferably of about equimolar.

Alternatively, the Cinacalcet Hydrochloride solvate with terachloromethane of the invention, when analyzed by PXRD, displays a spectrum comprising peaks at 2 theta angles of about 8.3 ± 0.2°, 14.8 ± 0.2°, 16.7 ± 0.2°, 20.2 ± 0.2°, 21.9 ± 0.2°, 22.9 ± 0.2° and 25.3 ± 0.2°.

Alternatively, the Cinacalcet Hydrochloride solvate with terachloromethane of the invention, when analyzed by IR, displays a spectrum with characteristic bands at about 3221, 2966, 2753, 1453, 1329, 1169, 1128, 1074, 800, 781 and 704 cm-1.

In a preferred embodiment, the invention relates to a solvate of Cinacalcet Hydrochloride with terachloromethane having a ratio of chloroform to Cinacalcet Hydrochloride of between 0.8 and 1.2, and comprising peaks in a PXRD spectrum at 2 theta angles of 16.7 ± 0.2° and 8.3 ± 0.2, and preferably also at 22.9 ± 0.2° and 14.8 ± 0.2°.

The solvate with terachloromethane may be prepared e.g. from a hot saturated solution in terachloromethane.

The terachloromethane solvate of Cinacalcet Hydrochloride can be used for the purification of Cinacalcet Hydrochloride and for the preparation of new polymorphic form II of Cinacalcet hydrochloride, for example by desolvation of the solvate.

The X-ray diffraction patterns were obtained using a Siemens D-5000 diffractometer (Bruker AXS, Karlsruhe, D) equipped with a theta/theta goniometer, a CuKα radiation source, a Goebel mirror (Bruker AXS, Karlsruhe, D), a 0.15° soller slit collimator and a scintillation counter. The patterns were recorded at a tube voltage of 40 kV and a tube current of 35 mA, applying a scan rate of 0.005° 2θs-1 in the angular range of 2 to 40° 2θ. Form II and form III were recorded at ambient temperature whereas form I was recorded at 160°C. X-axis is the 2-Theta scale, Y-axis gives the counts per second.

DSC curves were recorded with a DSC 7 system (Perkin Elmer, Norwalk, Ct.,USA) using the Pyris 2.0. software. Samples of approximately 2 mg were weighted into A1 pans with perforated cover. Dry nitrogen was used as purge gas. X-axis is the temperature in °C, Y-axis gives dH/dT (endo up).

### Description of the Figures:

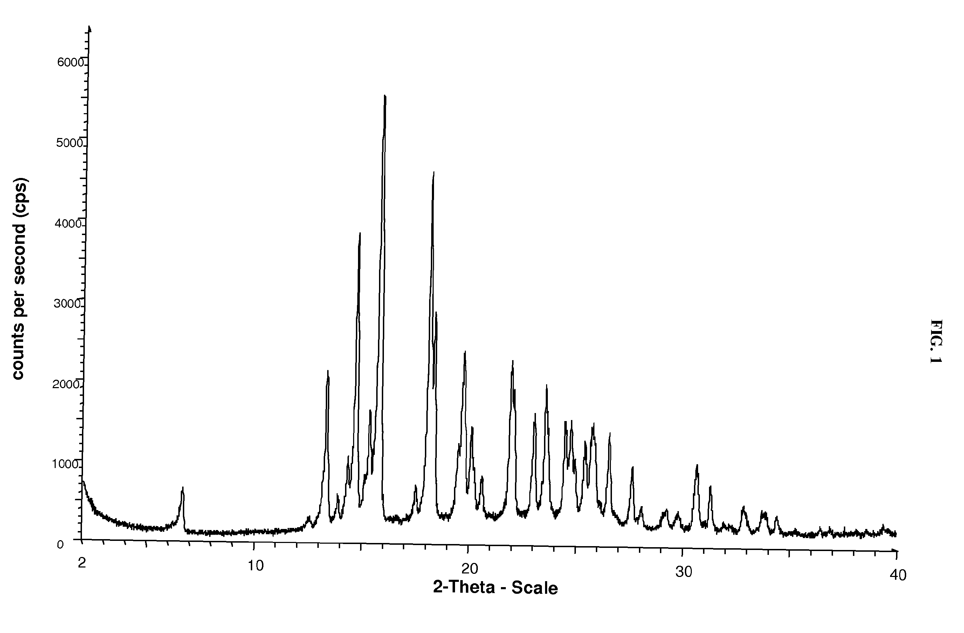

Figure 1: PXRD of crystalline Form I of Cinacalcet

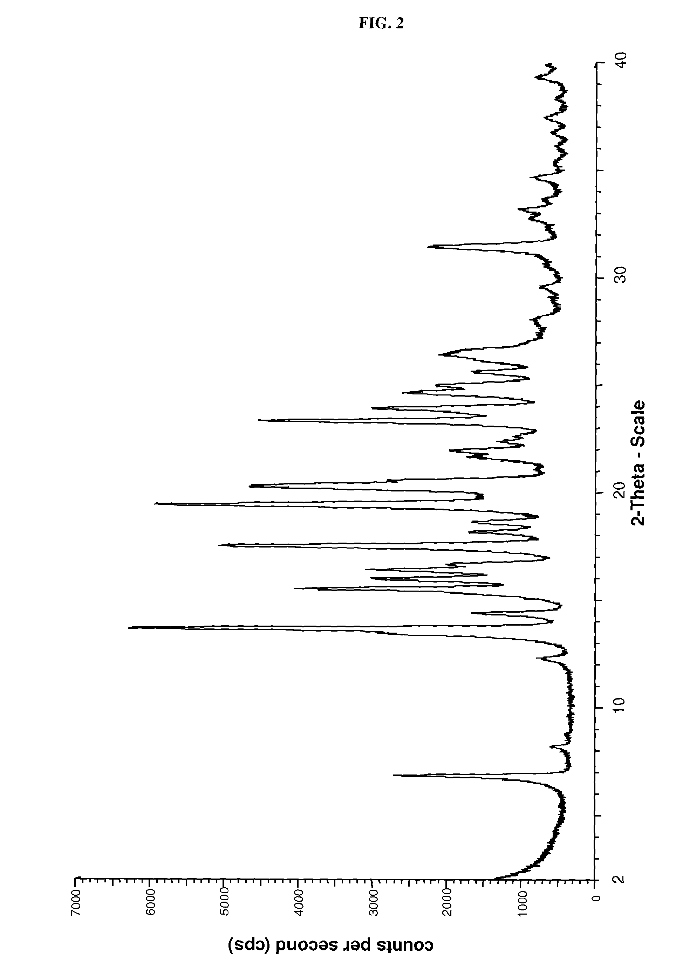

Figure 2: PXRD of crystalline Form II of Cinacalcet

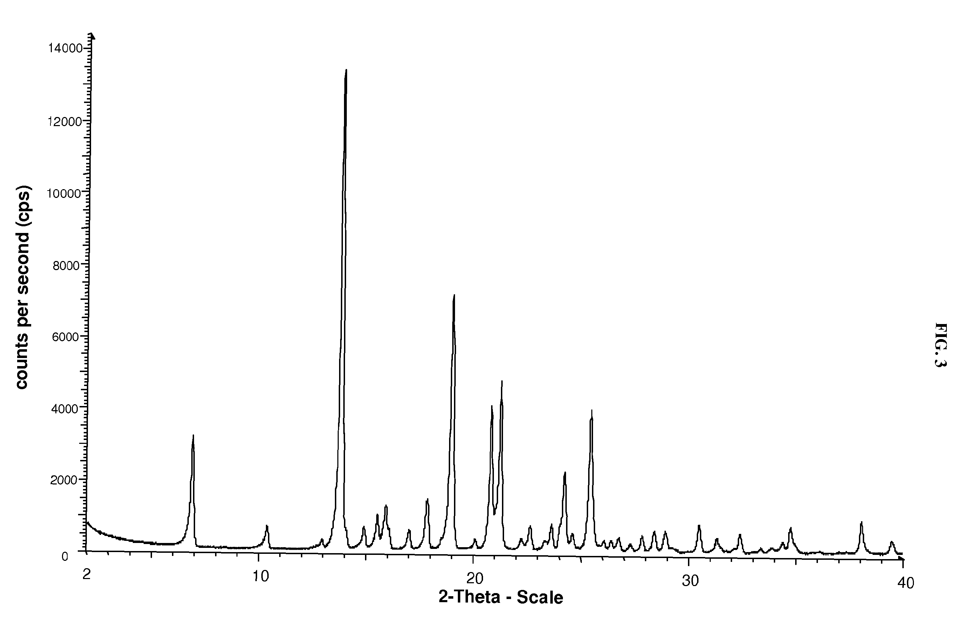

Figure 3: PXRD of crystalline Form III of Cinacalcet

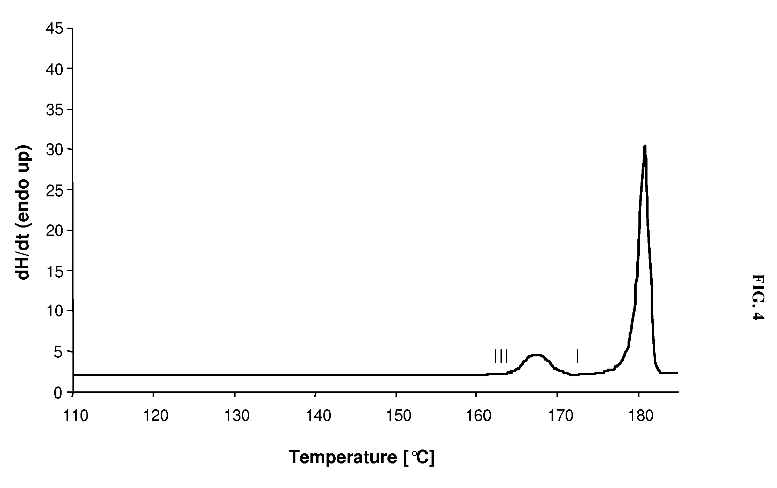

Figure 4: DSC of form III of Cinacalcet (heating rate: 10 k min-1)
   The DSC of form III of Cinacalcet shows an exothermic peak with onset of about 164°C due to transformation of form I . Form I is a crystalline form which melts at about 180°C and is only stable at temperatures above 130°C.

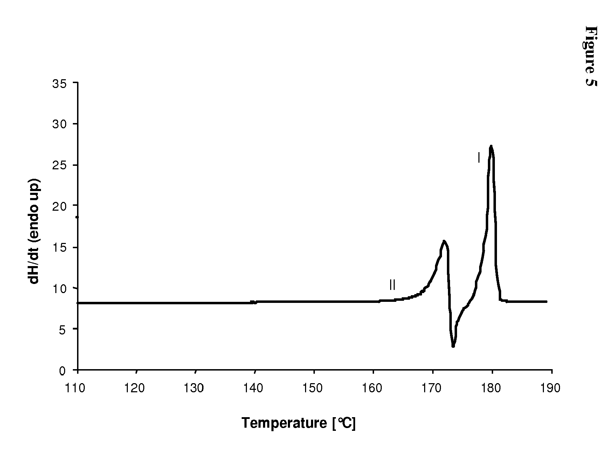

Figure 5: DSC of form II of Cinacalcet hydrochloride (heating rate: 10 k min-1)
   The DSC curve of form II shows an inhomogeneous melting process starting at about 170°C followed by recrystallization to form I, which is only stable at temperatures above about 130°C.

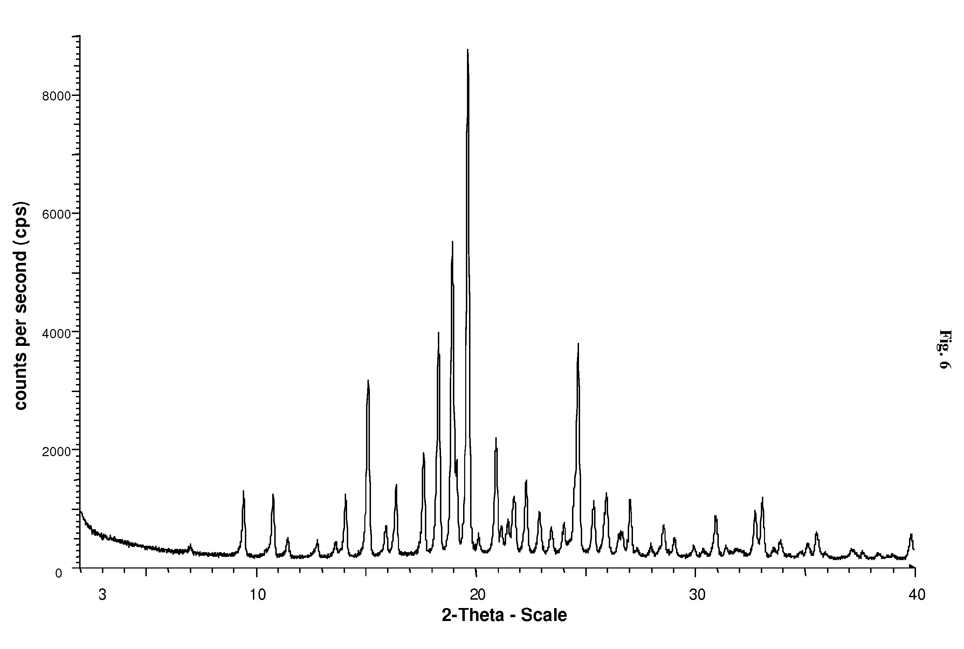

Figure 6 : PXRD of the solvate of Cinacalcet with acetic acid

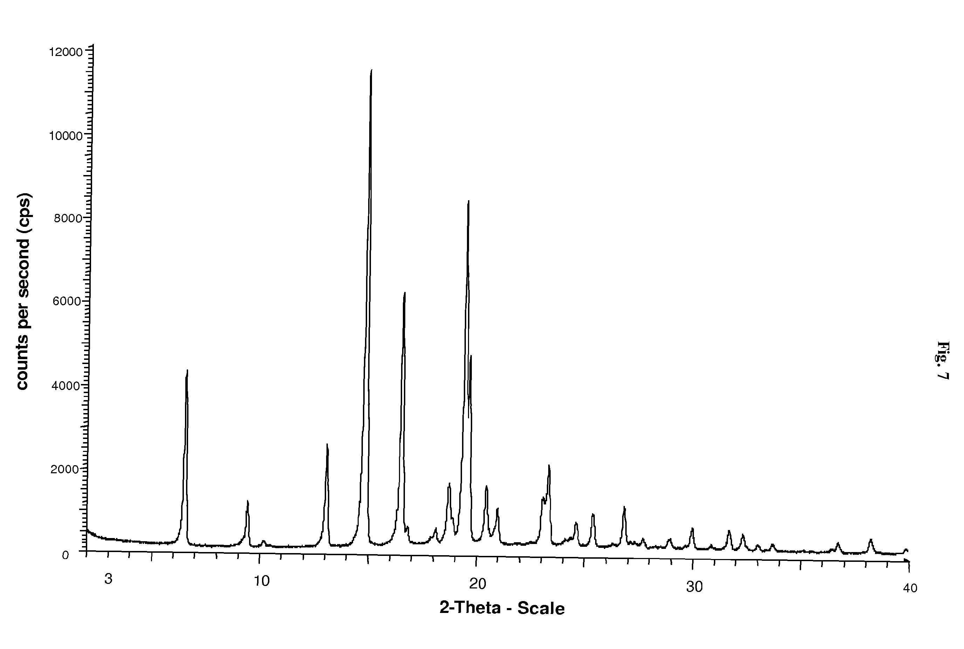

Figure 7: PXRD solvate of Cinacalcet with dioxane

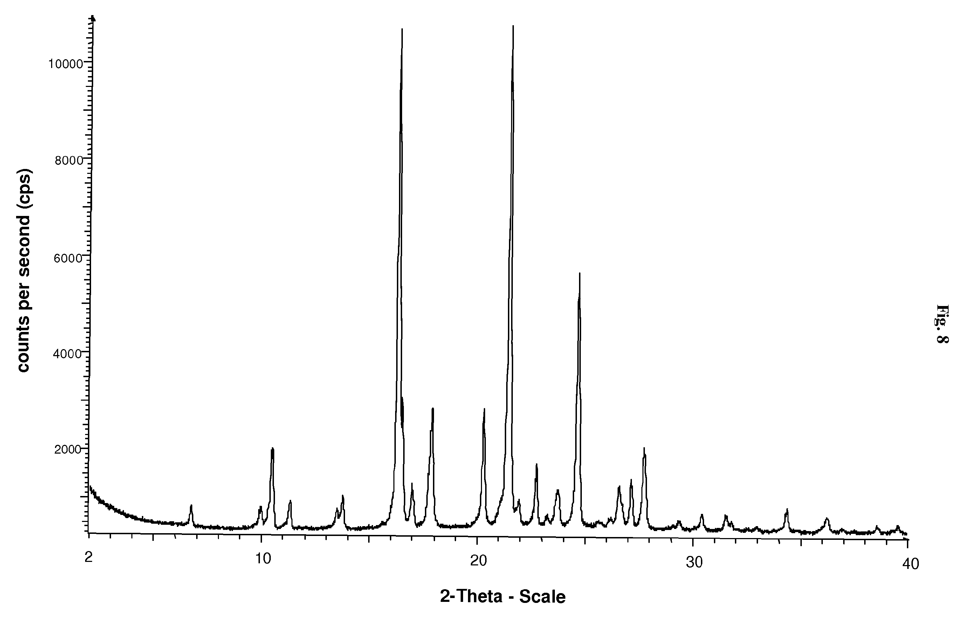

Figure 8: PXRD solvate of Cinacalcet with chloroform

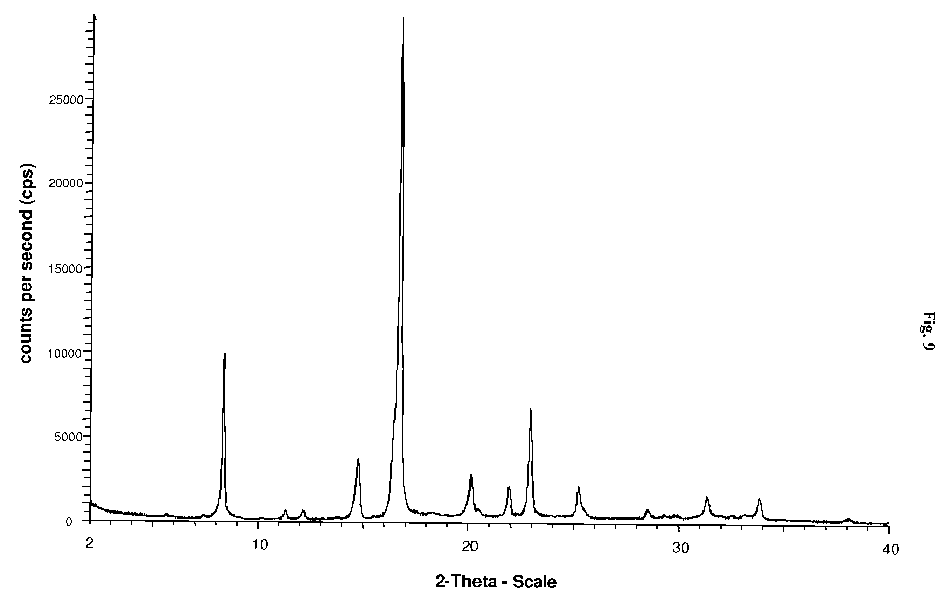

Figure 9: PXRD of the solvate of Cinacalcet with TCM

### Examples

The following examples describe the present invention in detail and are not to be construed to be in any way limiting to the scope of the claims.

### Example 1

107.1 mg of Cinacalcet hydrochloride was dissolved in 0.5 ml 2-PrOH at 90°C. The solution was cooled to 0°C in an ice bath, filtrated and dried at reduced pressure (20mbar) at ambient temperatures to yield form III of Cinacalcet hydrochloride in the form of needles.
Yield: 73.0 mg

### Example 2

102.5 mg of Cinaclacet hydrochloride was dissolved in 0.5 ml 1-PrOH at reflux. Seeds of Cinacalcet form III (prepared by example 1) were added and the suspension was cooled to room temperature within one hour, filtrated and dried at reduced pressure (20 mbar) at ambient temperatures yield form III of Cinacalcet Hydrochloride in the form of plates.
Yield: 67.9 mg

### Example 3:

10 g of Cinacalcet hydrochloride in 90 ml of ethylacetate were dissolved by heating the suspension to the boiling point. The solution was allowed to cool to ambient temperature within approximately 2 hours. The crystals were collected by filtration and dried in vacuo at ambient temperature yield form III of Cinacalcet in the form of needles.
Yield: 7.73 g.

In analogy to examples 1 to 3 form III of Cinacalcet hydrochloride was prepared using the solvent and solvent mixtures given in table 1

### General Procedure:

A saturated solution of Cinacalcet hydrochloride at the boiling point of the solvent or solvent mixture was prepared followed by cooling. In case of a second solvent twice the amount of the solvent showing lower solubility was used.

**Table 1**

| Solvent | Form | |
|---|---|---|
| Acetic acid | | |
| + water | III | Needles |
| Acetone | III | |
| + water | III | Needles |
| + hexane | III | Needles and plates |
| Acetonitrile | III | Plates |
| + water | III | Plates |
| 1-BuOH | III | Needles and plates |
| + hexane | III | Plates |
| Dichloroethylene | III | Needles |
| Dichloromethane | III | Needles |
| + water | III | Needles |
| + hexane | III | Needles |
| Diethylether | III | Needles |
| Dioxan | Solvate | Plates |
| + water | III | Plates |
| DMF | III | Plates |
| + water | III | Plates |
| DMSO | III | Plates |
| + water | III | Plates |
| Ethylmethylketone | III | Plates |
| + hexane | III | Needles |
| EtOH | III | Plates |
| + water | III | Plates |
| + hexane | III | Plates |
| Ethylacetate | III | Needles |
| MeOH | III | Plates |
| + water | III | Plates |
| Methylacetate | III | Needles |
| Nitromethane | III | Plates |
| 1-PrOH | III | Plates |
| + water | III | Needles, plates |
| 2-PrOH | III | Needles |
| + water | III | Needles |
| Propionic acid | III | Needles |
| Pyridine | III | Needles, plates |
| THF + water | III | Needles |
| Toluene | III | Needles |
| + heptane | III | Needles,plates |
| Water | III | Needles, plates |
| Xylene | III | Needles |

### Example 4:

100.8 mg of Cinacalcet hydrochloride was dissolved in 0.5 ml of methanol at room temperature. In a second step 1.0 ml of water was added to decrease the solubility. Cinacalcet precipitated. Cinacalcet form III was filtrated and dried at reduced pressure (20 mbar) at ambient temperatures.
Yield: 75.9 mg

In analogy to example 4 form III of Cinacalcet was prepared using the solvents given in table 2.

### General Procedure:

A saturated solution of Cinacalcet hydrochloride at room temperature was produced. The second solvent was added till crystallization occurred (about twice the amount of the first solvent). Optionally seeds of form III were added prior to the addition of the second solvent.

**Table 2:**

| Solvent | Antisolvent | Form | |
|---|---|---|---|
| Acetic acid | Water | III | Needles |
| Acetone | Water | III | Needles |
| Acetonitrile | Water | III | Plates |
| BuOH | Heptane | III | Plates |
| DMF | Water | III | Needles |
| DMSO | Water | III | Needles, plates |
| Dioxan | Water | III | Plates |
| EtOH | Water | III | Plates |
| MeOH | Water | III | Needles |
| 1-PrOH | Water | III | Needles, plates |
| 2-PrOH | Water | III | Plates |
| THF | Water | III | Needles |

### Example 5:

To a solution of 1.15 g Cinacalcet free base in 5 ml of acetone was added 0.5 ml HCl conc. followed by drop wise addition of 10 ml of water. Seeds of Cinacalcet hydrochloride were added when the solution became turbid. The suspension was stirred for 1 hour at ambient temperature and the product was isolated by filtration and dried in vacuo at ambient temperature overnight to yield form III of Cinaclcet Hydrochloride.
Yield: 0.83 g
PXRD of form III of Cinacalcet hydrochloride

| 2-Theta (°) | d (?) |
|---|---|
| 6.9 | 12.87 |
| 10.3 | 8.54 |
| 12.9 | 6.85 |
| 13.8 | 6.41 |
| 14.9 | 5.95 |
| 17.0 | 5.22 |
| 17.8 | 4.97 |
| 19.0 | 4.68 |
| 20.8 | 4.27 |
| 21.2 | 4.18 |
| 22.6 | 3.93 |
| 24.2 | 3.67 |
| 25.4 | 3.50 |

### Example 6

### Preparation of a mixture of form II and form III of Cinacalcet hydrochloride

554 mg of Cinacalc et hydrochloride was dissolved in a 1:1 mixture of dichloromethane and heptane (each 20 ml) at approximate 65°C. The solvent was removed at reduced pressure (200 mbar) at 40°C. Cinacalcet was dried at reduced pressure to give a mixture of form II and form III of Cinacalcet hydrochloride.
Yield: 521 mg

### Example 7

### Acetic acid solvate of Cinacalcet hydrochloride

130.6 mg of Cinacalcet hydrochloride was dissolved in 0.35 ml of acetic acid at 120°C. The solution was allowed to cool to ambient temperature and filtered, and dried at reduced pressure (20 mbar) at ambient temperature.
Yield: 130 mg of acetic acid solvate
PXRD:

| 2-Theta (°) | d (?) | Rel. Intensity (%) |
|---|---|---|
| 6.98 | 12.66 | 4.4 |
| 9.40 | 9.40 | 14.8 |
| 10.43 | 8.48 | 3.6 |
| 10.75 | 8.22 | 14.2 |
| 11.42 | 7.74 | 5.7 |
| 12.75 | 6.94 | 5 |
| 13.61 | 6.50 | 4.7 |
| 14.06 | 6.30 | 14.1 |
| 15.09 | 5.87 | 36.2 |
| 15.89 | 5.57 | 7.9 |
| 16.35 | 5.42 | 16.1 |
| 17.62 | 5.03 | 22.2 |
| 18.30 | 4.84 | 45.6 |
| 18.94 | 4.68 | 63.4 |
| 19.64 | 4.52 | 100 |
| 20.13 | 4.41 | 6.5 |
| 20.93 | 4.24 | 25.2 |
| 21.18 | 4.19 | 8 |
| 21.46 | 4.14 | 9.3 |
| 21.75 | 4.08 | 13.7 |
| 22.30 | 3.98 | 16.8 |
| 22.91 | 3.88 | 10.8 |
| 23.45 | 3.79 | 7.8 |
| 24.02 | 3.70 | 8.2 |
| 24.66 | 3.61 | 43.5 |
| 25.37 | 3.51 | 13 |
| 25.97 | 3.43 | 14.4 |
| 26.67 | 3.34 | 6.7 |
| 27.05 | 3.29 | 13 |
| 27.36 | 3.26 | 3.7 |
| 28.01 | 3.18 | 4.6 |
| 28.58 | 3.12 | 8.3 |
| 29.07 | 3.07 | 5.6 |
| 29.95 | 2.98 | 4.2 |
| 30.4 | 2.94 | 3.6 |
| 30.96 | 2.89 | 9.9 |
| 31.43 | 2.84 | 3.8 |
| 31.90 | 2.80 | 3.3 |
| 32.76 | 2.73 | 10.8 |
| 33.08 | 2.71 | 13.5 |
| 33.61 | 2.66 | 3.7 |
| 33.92 | 2.64 | 5.2 |
| 34.84 | 2.57 | 3 |
| 35.17 | 2.55 | 4.7 |
| 35.57 | 2.52 | 6.7 |
| 35.96 | 2.50 | 2.7 |
| 37.19 | 2.42 | 3.4 |
| 37.67 | 2.39 | 2.8 |
| 38.38 | 2.34 | 2.8 |
| 39.04 | 2.31 | 2.5 |

The TGA shows a mass loss of 13.1 % corresponding to 0.99 mol of acetic acid

### Example 8

### Preparation of form II of Cinacalcet hydrochloride

35.0 mg of Cinacalcet hydrochloride acetic acid solvate from example 7 was dried in a drying oven at 140°C for 30 min.
29.5 mg Form II of Cinacalcet hydrochloride was obtained.

PXRD data of form II of Cinacalcet:

| 2-Theta (°) | D (?) |
|---|---|
| 6.8 | 13.01 |
| 8.1 | 10.85 |
| 12.3 | 7.21 |
| 13.7 | 6.48 |
| 14.4 | 6.16 |
| 16.4 | 5.41 |
| 17.5 | 5.06 |
| 18.2 | 4.88 |
| 19.4 | 4.57 |
| 20.3 | 4.38 |
| 21.9 | 4.05 |
| 23.3 | 3.81 |
| 23.9 | 3.72 |

### Example 9

### Dioxane solvate of Cinacalcet hydrochloride

A solution of 118 mg of Cinacalcet hydrochloride was prepared by heating 0.65 ml dioxane at 90°C . The solution was allowed to cool to ambient temperature and dried at reduced pressure (20 mbar) at ambient temperature.
Yield: 99 mg
PXRD:

| 2-Theta (°) | d(?) | Rel Intensity (%) |
|---|---|---|
| 6.49 | 13.60 | 36.9 |
| 9.39 | 9.41 | 10.7 |
| 10.16 | 8.70 | 2.5 |
| 13.05 | 6.78 | 22.5 |
| 14.84 | 5.97 | 100 |
| 16.52 | 5.36 | 54 |
| 16.8 | 5.27 | 5.7 |
| 18.10 | 4.90 | 5.5 |
| 18.72 | 4.74 | 14.8 |
| 19.43 | 4.57 | 72.4 |
| 19.63 | 4.52 | 41.1 |
| 20.44 | 4.34 | 14.4 |
| 20.97 | 4.23 | 9.8 |
| 23.09 | 3.85 | 12 |
| 23.32 | 3.81 | 18 |
| 24.14 | 3.68 | 3.6 |
| 24.62 | 3.61 | 7.1 |
| 25.40 | 3.51 | 8.8 |
| 26.34 | 3.38 | 2.6 |
| 26.85 | 3.32 | 10.5 |
| 27.73 | 3.22 | 3.7 |
| 28.95 | 3.08 | 3.8 |
| 29.99 | 2.98 | 6 |
| 30.89 | 2.89 | 2.8 |
| 31.71 | 2.82 | 5.8 |
| 32.36 | 2.77 | 4.9 |
| 33.05 | 2.71 | 2.9 |
| 33.73 | 2.66 | 2.9 |
| 36.48 | 2.46 | 2.2 |
| 36.76 | 2.44 | 3.4 |
| 38.28 | 2.35 | 4.3 |

### TGA shows a mass loss 16.1 % corresponding to 0.86 mol of dioxane

Example 10 Chloroform solvate of Cinacalcet hydrochloride
108 mg of Cinacalcet hydrochloride was dissolved in 0.45 ml of chloroform at approximately 65°C.

The solution was allowed to cool to ambient temperature. The product was isolated by filtration and dried at reduced pressure (20 mbar) at ambient temperature.
Yield: 108 mg
PXRD:

| 2-Theta (°) | d (?) | Rel. Intensity (%) |
|---|---|---|
| 6.70 | 13.18 | 10.6 |
| 9.93 | 8.90 | 10.4 |
| 10.45 | 8.46 | 21 |
| 11.30 | 7.83 | 11.6 |
| 13.48 | 6.56 | 10.1 |
| 13.74 | 6.44 | 12.3 |
| 16.32 | 5.43 | 99.3 |
| 16.98 | 5.22 | 15 |
| 17.87 | 4.96 | 28.6 |
| 20.29 | 4.37 | 28.8 |
| 21.49 | 4.13 | 100 |
| 21.92 | 4.05 | 11.7 |
| 22.73 | 3.91 | 16.9 |
| 23.25 | 3.82 | 9.1 |
| 23.75 | 3.74 | 13.9 |
| 24.67 | 3.61 | 53.3 |
| 25.65 | 3.47 | 8.2 |
| 26.21 | 3.40 | 8.9 |
| 26.60 | 3.35 | 14.6 |
| 27.18 | 3.28 | 15.1 |
| 27.77 | 3.21 | 21.9 |
| 29.41 | 3.04 | 8.3 |
| 30.46 | 2.93 | 9.6 |
| 31.57 | 2.83 | 9.4 |
| 31.83 | 2.81 | 8.4 |
| 32.99 | 2.71 | 7.3 |
| 34.39 | 2.61 | 10.5 |
| 36.27 | 2.48 | 9.1 |
| 38.61 | 2.33 | 7.6 |
| 39.57 | 2.28 | 7.7 |

TGA shows a mass loss of 22.8% corresponding to 0.98 mol of chloroform

### Example 11

Preparation of the tetrachloromethane solvate of Cinacalcet hydrochloride
121 mg of Cinacalcet hydrochloride was dissolved in 2.0 ml of tetrachloromethane at approximately 80°C. The solution was allowed to cool to ambient temperature. The product was isolated by filtration and dried at reduced pressure (20 mbar) at ambient temperature.
Yield: 132 mg
PXRD of tetrachloromethane solvate of Cinacalcet

| 2-Theta (°) | d (?) | Rel. Intensity (%) |
|---|---|---|
| 2.25 | 39.24 | 2.9 |
| 5.63 | 15.69 | 1.3 |
| 6.73 | 13.12 | 0.6 |
| 7.40 | 11.93 | 1 |
| 8.33 | 10.60 | 33.1 |
| 9.07 | 9.74 | 0.8 |
| 10.17 | 8.70 | 0.6 |
| 11.30 | 7.83 | 2.2 |
| 12.15 | 7.28 | 2.2 |
| 13.79 | 6.42 | 0.9 |
| 14.76 | 5.60 | 12.4 |
| 15.51 | 5.71 | 1.2 |
| 16.73 | 5.30 | 100 |
| 17.50 | 5.06 | 2.1 |
| 18.32 | 4.84 | 1.9 |
| 18.95 | 4.68 | 1.5 |
| 20.16 | 4.40 | 9.6 |
| 20.46 | 4.34 | 2.7 |
| 21.92 | 4.05 | 7.1 |
| 22.94 | 3.87 | 22.6 |
| 23.95 | 3.71 | 1.5 |
| 25.25 | 3.52 | 6.9 |
| 25.55 | 3.48 | 2.5 |
| 28.55 | 3.12 | 2.7 |
| 29.36 | 3.04 | 1.6 |
| 29.86 | 2.99 | 1.6 |
| 31.39 | 2.85 | 5.2 |
| 32.04 | 2.79 | 1.6 |
| 32.59 | 2.75 | 1.7 |
| 33.16 | 2.70 | 1.8 |
| 33.87 | 2.65 | 5 |
| 38.14 | 2.36 | 1.3 |

## Claims

1. Crystalline form II of Cinacalcet hydrochloride with a PXRD comprising peaks at 2 Theta angles of 6.8 ±0.2°, 12.3 ±0.2°, 13.7 ±0.2°, 16.4 ±0.2°, 17.5 ±0.2, 19.4 ±0.2, 20.3 ±0.2 and 23.3 ±0.2.

2. Pharmaceutical composition comprising Cinacalcet hydrochloride form II according to claim 1.

3. A process for the preparation of pure form II according to claim 1, comprising the step of desolvating a solvate of Cinacalcet hydrochloride.

4. Crystalline form III of Cinacalcet hydrochloride with a PXRD comprising peaks at 2 Theta angles of 6.9±0.2°, 10.3 ±0.2°, 13.8 ±0.2°, 19.0 ±0.2, 20.8 ±0.2, 21.2 ±0.2, 24.2 and 25.4 ±0.2.

5. The crystalline form III according to claim 4 in the form of plates.

6. Pharmaceutical composition comprising Cinacalcet hydrochloride form III according to any one of claims 4 and 5, preferably wherein Cinacalcet hydrochloride form III is present in a pharmaceutically effective amount.

7. The pharmaceutical composition according to claim 6, wherein at least 90% by weight of the comprised Cinacalcet hydrochloride is in the form of Cinacalcet hydrochloride form III.

8. The pharmaceutical composition according to any one of claims 6 and 7, wherein at least 60% of the Cinacalcet hydrochloride crystals are in the form of plates, preferably at least 80%, more preferably at least 90%.

9. A process for the preparation of pure form III of Cinacalcet hydrochloride comprising the steps of
a) providing a solution of Cinacalcet hydrochloride in a solvent selected from the list consisiting of acetone, methylethylketone, methylisobutylketone, 1-butanol, 2-butanol, ethanol, 3-methyl-1-butanol, isobutylalcohol, n-propanol, 2-propanol, 1-pentanol, butylacetate, ethylacetate, ethylformate, isobutylacetate, isopropylacetate, methylacetate, propylacetate, t-butylmethylether, diethylether, diisopropylether, anisol, dimethylsulfoxide, N-methylpyrrolidone, acetonitrile, dichloromethane, 1,2-dimethoxyethane, N,N-dimethylacetamide, N,N-dimethylformamide, n-hexane, methylcyclohexane, methanole, 2-methoxyethanole, 2-ethoxyethanole, methylbutylketone, xylene, 1,1-diethoxypropane and 1,1-dimethoxymethane, or mixtures of these solvents or mixtures of these solvents,with , n-hexane, methylcyclohexane, n-pentane, cyclohexane or water or mixtures of formic acid or acetic acid or tetrahydrofurane and water.
b) allowing pure form III of Cinacalcet hydrochloride to crystallize, and
c) isolating the pure crystalline form III of Cinacalcet hydrochloride.

10. The process according to claim 9 for the preparation of pure form III of Cinacalcet hydrochloride in the form of plates, wherein the solvent is selected from the list consisiting of ethylmethylketone, ethanole, and n-propanole, or mixtures of these solvents with water.

11. Cinacalcet Hydrochloride solvate with Dioxane, with an XPRD spectrum comprising peaks at 2 theta angles of about 6.5 ± 0.2°, 13.0 ± 0.2°, 14.8 ± 0.2°, 16.5 ± 0.2°, 19.4 ± 0.2°, 19.6± 0.2°.

12. Cinacalcet Hydrochloride solvate with acetic acid, with an XRPD spectrum comprising peaks at 2 theta angles of about 15.1 ± 0.2°, 17.6 ± 0.2°, 18.3 ± 0.2°, 18.9 ± 0.2°, 19.6 ± 0.2°, 20.9 ± 0.2°, 24.7 ± 0.2°.

13. Cinacalcet Hydrochloride solvate with chloroform, with an XRPD spectrum comprising peaks at 2 theta angles of about 16.3 ± 0.2°, 17.9 ± 0.2, 20.3 ± 0.2 , 21.5 ± 0.2, 24.7 ± 0.2, 27.8± 0.2°.

14. Cinacalcet Hydrochloride solvate with tetrachloromethane, with an XRPD spectrum comprising peaks at 2 theta angles of about 8.3 ± 0.2°, 14.8 ± 0.2°, 16.7 ± 0.2°, 20.2 ± 0.2°, 21.9 ± 0.2°, 22.9 ± 0.2° and 25.3 ± 0.2°.
